# EUROPEAN PATENT APPLICATION

(11) **EP 4 835 073 A1**
(43) Date of publication of application: **04.11.2026**
(21) Application number: 24909256.0
(22) Date of filing: 27.12.2024
(51) Int. Cl.: C12N 15/113, A01N 63/60, A01P 7/04, C12N 15/82

(54) **DSRNA MOLECULES, SMALL INTERFERING RNA (SIRNA) MOLECULES, COMPOUND, METHOD OF OBTAINING DSRNA MOLECULES, PLASMID, HOST CELL, PEST CONTROL METHOD**

(30) Priority: 29.12.2023 US 202363616223 P
(71) Applicant: Sempre Agtech Ltda., 89.802-210 Chapecó - SC (BR); Empresa Brasileira De Pesquisa Agropecuária - EMBRAPA, 70770-901 Brasília - Df (BR)
(72) Inventor: MOLINARI, Hugo Bruno Correa, 89.802-210 Chapecó - SC (BR); DE FARIAS, Marcelo Picanço, 89.802-210 Chapecó - SC (BR); MOLINARI, Mayla Daiane Correa, 89.802-210 Chapecó - SC (BR); ABBAD, Samantha Vieira, 89.802-210 Chapecó - SC (BR); ROGGIA, Isabel, 86.047-490 Londrina - PR (BR); DA ROSA, Juliana, 86.015-090 Londrina - PR (BR); KOBAYASHI, Adilson Kenji, 70.770-901 Plano Piloto - DF (BR); NEPOMUCENO, Alexandre Lima, 70.770-901 Plano Piloto - DF (BR); VIANA, Américo José Carvalho, 70.770-901 Plano Piloto - DF (BR); HENNING, Liliane Marcia Mertz, 70.770-901 Plano Piloto - DF (BR); FRAGOSO, Rodrigo Da Rocha, 70.770-901 Plano Piloto - DF (BR); MARIN, Silvana Regina Rockenbach, 70.770-901 Plano Piloto - DF (BR)
(74) Representative: Vossius & Partner Patentanwälte Rechtsanwälte mbB
(86) International application number: PCT/BR2024/050604
(87) International publication number: WO 2025/137756

(57) **Abstract**

The present invention refers to double-stranded RNA (dsRNA) molecules, compounds comprising dsRNA molecules, method of obtaining dsRNA molecules, and phytosanitary control methods in soybean-corn production system. The compound comprising dsRNA molecules of the present invention has an efficient and specific mode of action against insect pests because it is biodirected, particularly in the control of brown stink bugs *(Euschistus heros*) and green-bellied stink bugs (*Diceraeus melacanthus*), being sustainable, in addition to being naturally non-toxic, non-allergenic and non-virulent.

## Description

### TECHNICAL FIELD

The present invention refers to double-stranded RNA (dsRNA) molecules, compounds comprising dsRNA molecules, method of obtaining dsRNA molecules, and methods of phytosanitary control in agricultural production systems, particularly in the soybean-corn production system. The compound comprising dsRNA molecules of the present invention has an efficient and specific mode of action against insect pests because it is biodirected, particularly in the control of brown stink bugs (*Euschistus heros*) and green-bellied stink bugs (*Diceraeus melacanthus*), being sustainable, in addition to being naturally non-toxic, non-allergenic and non-virulent.

### BACKGROUND OF THE INVENTION

The production of food to serve the world's population represents one of the greatest challenges in the world. With a population that is still growing, and which is expected to reach 10 billion people by 2057, producing food efficiently is one of the great and pressing global issues.

In this sense, agriculture plays a fundamental role, and today it is one of the economic activities that most demands innovation and new technological solutions. The search for increasing productivity and profitability of crops in the world, and on a large scale in Brazil - one of the breadbaskets of the planet - is a constant objective. Within this purpose, one of the fields in which there is a need for frequent evolution is that of the control of pest insects of cultivated crops. Whether because of issues related to pest resistance to be controlled, or because of the need to improve the impacts of pesticides to increase the country's productive potential, or even all the reasons mentioned.

Today, the search for a cleaner, more modern, sustainable agriculture that results in increased productivity is the keynote of its technological development.

Specifically in relation to the control of pest insects in the agricultural production system, such as in the soybean-corn production system, we have a relevant problem, which is the issue of stink bugs. The family *Pentatomidae* is the fourth largest family of the suborder *Heteroptera* and comprises more than 36 thousand species, with 760 genera and 4,100 species having been described in Brazil (Froeschner et al., 2019). The pentatomid stink bug complex has great economic importance in the agricultural scenario due to its polyphagous feeding habit and for causing relevant damage to the attacked crops, causing a reduction in yield and quality (Corrêa-Ferreira et al., 2002). Among the stink bugs of this complex, two species have stood out in the agricultural production system, particularly in the soybean-corn production system, the brown stink bug (*Euschistus heros*) and the green-bellied stink bug (*Diceraeus melacanthus*). The brown stink bug is considered a key pest in soybean crops mainly due to its wide distribution and frequency of occurrence of 70% among the stink bugs that attack this crop, and can cause losses of up to 90% in the productivity of this crop if not properly treated, and the presence of just one stink bug can reduce productivity by up to nine kilos per ha/day (Panizzi, 2016; Panizzi et al., 2022). The green-bellied stink bug, on the other hand, has caused significant and increasing losses in the corn crop (Chocorosqui; Panizzi, 2004). The control of these stink bugs with chemical insecticides has been shown to be less and less effective, due to the increased resistance of these insects to the main chemical molecules traditionally used (Somavilla, 2020).

Thus, the economic importance of controlling the brown stink bug and the green-bellied stink bug has been increasing in recent years, with brown being considered the main soybean pest in Brazil (Panizzi et al., 2022). It is estimated that around 40% of the chemical insecticides applied to soybeans are for the control of stink bugs (Tuelher et al., 2018). Thus, it is extremely necessary to develop a new alternative for the control of these stink bugs.

RNA interference (RNAi) technology is an excellent alternative to the use of chemical insecticides, as it has a restricted spectrum of target insects, avoiding effects on beneficial insects such as pollinators, for example, and also because it is a biodegradable molecule (Vogel et al., 2019). This natural biological process has enormous potential for applications in agriculture, including its use as a product based on dsRNA molecules, with insecticidal action, against specific genes of brown and green-bellied stink bugs.

This technology is based on silencing, via RNAi, the natural biological process of expression of a gene, a process observed in eukaryotic organisms. This process is activated by the presence of a double-stranded RNA (dsRNA) that targets a specific messenger RNA (mRNA) that, in turn, would be translated into a protein. Once inside the cell's cytoplasm, dsRNA is processed by RNAi's natural machinery, starting with its recognition by the DICER ribonuclease that cleaves it into smaller fragments of 19 to 22 nucleotides. These smaller fragments, called *small-interfering* RNA (siRNA), are then bound by the ARGONAUTA protein, generating the RNA-induced silencing complex (RISC) (Lee et al., 2004). After the formation of the RISC complex in the cytoplasm of the cell, the siRNA will bind to an mRNA that has a complementary sequence and promote its cleavage and degradation, culminating in the silencing of the expression of that gene (Okamura et al., 2004). The biological mechanism of RNAi is located entirely in the cytoplasm of the cell, so there is no risk of alteration in the nuclear DNA sequence.

This natural biological process has enormous potential for commercial applications, whether in health, industry or agriculture. In agriculture, efforts have been made to develop biotechnological solutions to obtain a dsRNA that acts as an active ingredient in the composition of biopesticides against insect pests, pathogens and weeds.

The document WO2018046312A2 discloses insecticidal compositions comprising a double-stranded ribonucleic acid, or dsRNA, in which these compositions are intended to regulate gene expression for gene silencing purposes.

However, there are still a number of challenges related to insecticidal compositions based on dsRNAs, especially with regard to pests that are harmful to the agricultural production system, such as the soybean-corn production system.

Thus, insecticidal compositions based on dsRNA molecules that represent new unique modes of action are necessary, in order to allow a marked mortality of brown and green-bellied stink bugs, both important pest insects of the agricultural production system, in addition to the aforementioned compositions not causing permanent modification of the genome of the plant in which they are applied, since they do not have recombinant DNA.

It is necessary to develop more sustainable biodirected pesticides, based on RNAi technology, so that a cleaner, more modern and sustainable agriculture is possible, with gains in productivity and profitability in plantations and crops, without necessarily increasing the cultivation area.

Therefore, the present invention refers to double-stranded RNA (dsRNA) molecules, compounds comprising dsRNA molecules, method of obtaining dsRNA molecules, plasmid, host cell and methods of controlling pest insects in agricultural production systems, particularly in soybean-corn production systems.

### SUMMARY OF THE INVENTION

The present invention discloses dsRNA molecules that contain the sequences for the expression of dsRNAs that activate the silencing of transcripts of the gene*s Trehalase II (TrehaII)*, *Trehalose Transporter II (TreTran II)* and *Transcription Initiation Factor TFIID Subunit 1 (TAF1)* of brown (*Euschistus heros*) and green-bellied (*Diceraeus melacanthus*) stink bugs, both pests in the agricultural production system, particularly in the soybean-corn production system, in addition to the dsRNA molecule that is characterized by being a concatamer comprising the combination between said dsRNA molecules.

One embodiment of the invention refers to compounds that comprise at least one of the dsRNA molecules described herein.

One embodiment of the invention refers to a method of obtaining dsRNA molecules that comprises the steps of:
a. cloning in cloning vector with instruction for production of dsRNA for a target gene;
b. producing via fermentation, transcription *in vitro*, chemical synthesis, biochemical synthesis or the combination thereof of an unpurified dsRNA;
c. purifying the dsRNA molecules obtained.

Another embodiment of the invention refers to a plasmid containing the synthetic sequences of the genes *Trehalase II*, *Trehalose Transporter II* and *Transcription Initiation Factor TFIID Subunit 1* that encode dsRNA sequences for silencing the genes of brown (*Euschistus heros*) and green-bellied (*Diceraeus melacanthus*) stink bugs.

The present invention also describes a host cell comprising the plasmid of the present invention.

In another embodiment of the invention, a method of pest control in an agricultural production system is described, which includes: spraying the compound comprising dsRNA molecules on the pest insects so that they come into contact with the aforementioned dsRNA molecules.

### BRIEF DESCRIPTION OF THE DRAWINGS

To assist in the identification of the main characteristics of this invention, some figures are presented to which reference is made, as follows:

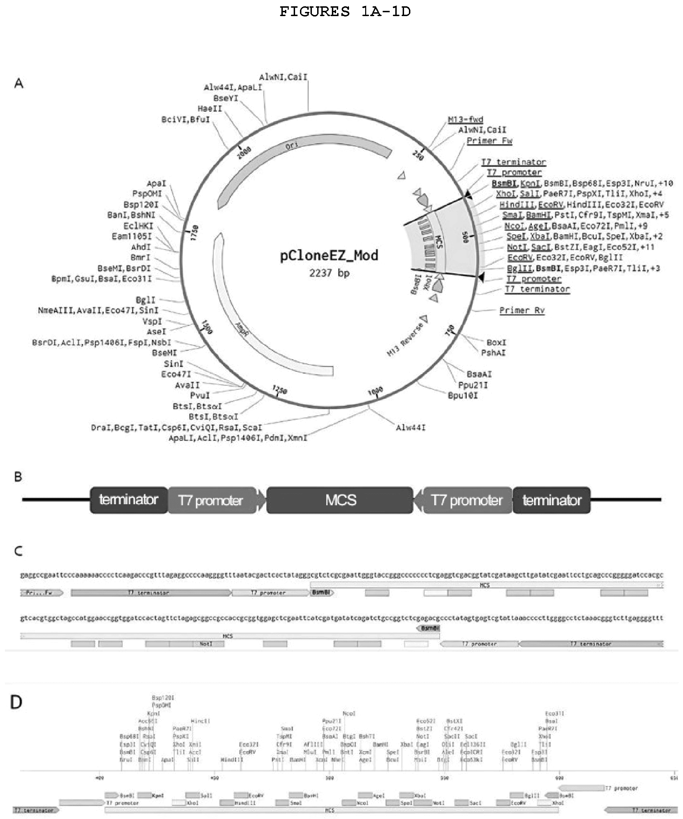

Figures 1a to 1d demonstrate the vector for dsRNA expression in *E. coli* HT115: a) Map of the modified pClone EZ vector to contain the region of the multiple cloning site and the bidirected T7 promoters and terminators, b) Schematic of the multiple cloning site and the bidirected T7 promoters and terminators contained in the modified pClone EZ vector, c) Nucleotide sequence of the multiple cloning site and the bidirected T7 promoters and terminators and the two restriction enzyme sites *BsmBI* contained in the modified EZ pClone vector. D) Linear map of the MCS of the modified pClone EZ vector showing the location of the restriction enzyme sites with emphasis on the two *BsmBI* sites positioned adjacent to the two T7 promoters. *MCS= Multiple Cloning Site.
Figure 2 represents Schematic of the HT115 strain of *E. coli* transformed with pClone EZ vector modified for heterologous expression of IPTG-inducible dsRNA. Modified from Klema et al. (2015).
Figure 3 shows the complete sequence of the modified pClone EZ plasmid.
Figure 4 shows the nucleotide sequence of the Beta-Lactamase gene contained in the pClone EZ vector and which confers resistance to the antibiotic ampicillin.
Figure 5 shows the amino acid sequence of the Beta-Lactamase gene contained in the pClone EZ vector and which confers resistance to the antibiotic ampicillin.
Figure 6 represents the general scheme of dsRNA molecule production in *E. coli* strain HT115.
Figure 7 represents the standard of integrity and purity of the dsRNA molecules *dsTreha II*, *dsTreTran II* and *dsTAF1* contained in compounds 001, 002 and 003 of the present invention, observed from electrophoresis in 1% agarose gel stained with ethidium bromide.
Figure 8 represents the solution of *dsTAF1* after purification, plated in LB medium with the antibiotic Tetracycline. Petri dish with and without lid indicating the absence of bacterial colony formation.
Figure 9 represents the solution of *dsTreTran II* after purification, plated in LB medium with the antibiotic Tetracycline. Petri dish with and without lid indicating the absence of bacterial colony formation.
Figure 10 depicts the solution of *dsTreha II* after purification, plated in LB medium with the antibiotic Tetracycline. Petri dish with and without lid indicating the absence of bacterial colony formation.
Figure 11 represents the solution of *E. coli* HT115 bacteria transformed with pClone EZ vector containing fragment *dsTAF1*, plated in LB medium with the antibiotic Ampicillin + Tetracycline. Petri dish with and without lid indicating the great growth of the colonies that formed "carpet".
Figure 12 represents unprocessed solution of *E. coli* HT115 bacteria, plated in LB medium with Ampicillin and Tetracycline antibiotics. Petri dish with and without lid indicating the absence of bacterial colony formation.
Figure 13 represents the unprocessed *E. coli* HT115 bacteria solution, plated in LB medium containing only the antibiotic Tetracycline. Petri dish with and without lid indicating the great growth of the colonies that formed "carpet".
Figure 14 represents the solution of *E. coli* HT115 transformed with pClone EZ vector containing fragment *dsTAF1*, plated in LB medium containing only the antibiotic Tetracycline. Petri dish with and without lid indicating the great growth of the colonies that formed "carpet".
Figure 15 shows 1% agarose gel electrophoresis with concentrations of 100, 500 and 1000 ng of the solution of the dsRNAs *Treha II*, *TreTran II* and *TAF1* stained with ethidium bromide.

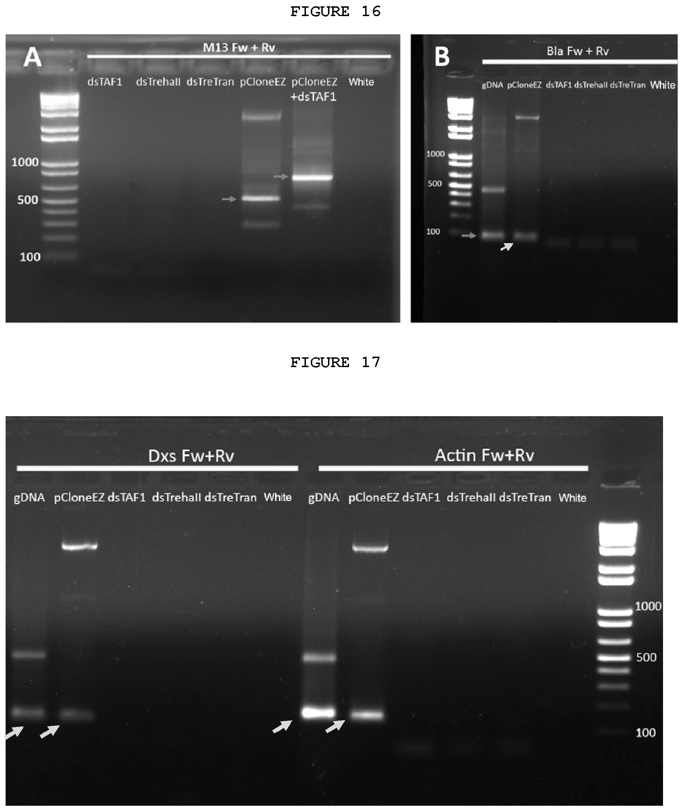

Figure 16 shows the 1% agarose gel electrophoresis of a PCR of the dsRNA solution of the present invention with specific primers for the pClone EZ vector. A) PCR reactions with M13 Fw + Rv primers. Channels 1, 2 and 3: using the solutions containing the dsRNAs *Treha II*, *TreTran II* and *TAF1* as a template: there was no amplification in any of the solutions; Channel 4: PCR with positive control pClone EZ (empty vector): there was an expected amplification of 515bp indicated by the red arrow; Channel 5: pClone EZ vector recombined with *dsTAF1*, used with second positive control: there was an expected amplification of 940bp; Channel 6: reaction blank (negative control); B) PCR reactions with Bla Fw + Rv primers. Channel 1: Genomic DNA of the bacterium used as a template: 81 bp sequence amplification, indicating the presence of the pClone EZ vector, as expected; Channel 2: positive control containing pClone vector EZ: 81 bp size amplicon, as expected; Channels 3, 4 and 5: templates were the final solution of the dsRNAs *Treha II*, *TreTran II* and *TAF1*: no amplification observed; Channel 6: negative control of the reaction. The gel was stained with ethidium bromide.
Figure 17 shows the PCR reactions of the dsRNA solutions of the present invention with specific primers for the chromosomal DNA of *E. coli*, with Dxs and Actin primers. Channels 1 and 7: positive control of the reaction using chromosomal DNA of the bacterium: amplification of 113 bp for the Dxs primer and 131 bp for the Actin primer, as expected; Channels 2 and 8: pClone EZ vector, with amplification of 113 bp for the Dxs primer and 131 bp for the Actin primer; Channels 3, 4 and 5: solutions of the dsRNAs *Treha II*, *TreTran II* and *TAF1* used as a template of the reactions with the primer Dxs: there was no amplification of the genomic DNA of the bacterium; Channels 9, 10 and 11: solutions of the dsRNAs *Treha II*, *TreTran II* and *TAF1* used as a template of the reactions with the primer Actin: there was no amplification of the genomic DNA of the bacterium; Channels 6 and 12: reaction blanks. Agarose gel electrophoresis 1%. The gel was stained with ethidium bromide.
Figure 18 shows 1% agarose gel electrophoresis of the digestion of the recombined, empty pClone EZ vector, using the restriction enzymes *BsmBI* and *XhoI*. Fragment of *TAF1* released after digestion (red arrow) confirms the specificity of cloning.
Figure 19 demonstrates the confirmation of the nucleotide sequences and alignment against the coding sequence (CDS) of the respective target genes of the dsRNAs *dsTreha II*, *dsTreTran II*, and *dsTAF1* of the present invention, contained in the compounds 001, 002 and 003 also described herein.
Figure 20 shows a graph of accumulated mortality on the 2nd and 15th day after microinjection for the brown stink bug.
Figure 21 shows a graph of accumulated daily mortality after microinjection for the brown stink bug.
Figure 22 shows a graph of the oviposition accumulated up to 15° after microinjection for the brown stink bug.
Figure 23 shows a graph of accumulated mortality on the 2nd and 15th day after microinjection for the green-bellied stink bug.
Figure 24 shows a graph of accumulated daily mortality after microinjection for the green-bellied stink bug.
Figure 25 shows a graph of the oviposition accumulated up to the 15th after microinjection for the green-bellied stink bug.
Figure 26 shows a graph of the mortality of *Euschistus heros* in a Potter's tower up to 7 days after dsRNA application (Drei)*naked*.
Figure 27 shows a graph of the mortality of *Diceraeus melacanthus* in a Potter's tower up to 7 days after dsRNA application (Drei)*naked*.

### DETAILED DESCRIPTION OF THE INVENTION

The present invention refers to double-stranded RNA (dsRNA) molecules, compounds comprising at least one of the dsRNA molecules, method of obtaining dsRNA molecules, and methods of pest control in agricultural production systems.

The present invention also refers to the dsRNA molecules that are characterized by being a concatamer comprising combinations between the aforementioned dsRNA molecules described herein.

The concatemer presents a characteristic totally different from all the genes already investigated to date for pest insects in agricultural production systems, with a very fast mortality effect comparable to or superior to the mode of action of a conventional insecticide.

For this, the present invention involves RNAi (RNA interference) technology, which is a natural cellular mechanism responsible for post-transcriptional gene silencing (PTGS). The RNAi acts on the target messenger RNA (mRNA), so that when a dsRNA molecule is incorporated into the cell, it activates a protein complex that culminates in the degradation of the target mRNA via binding to a complementary region in the target mRNA. This binding causes silencing by inhibition of the translation and/or degradation of the mRNA of the target organism.

Silencing elements may include, and are not limited to, a sensed suppression element, an antisense suppression element, a double-stranded RNA (dsRNA), a siRNA (small interfering RNA), an amiRNA, a miRNA, or a hairpin suppression element.

Silencing a gene via RNAi is a highly specific process, so that any mRNA in a cell can be intentionally degraded through it. In addition, once it is activated in an initial cell, the RNAi signal is transmitted to the other cells, exponentially increasing its effect. As a consequence, the high specificity of the RNAi mechanism makes the technology safe for the environment and for human and animal health, with no effect on non-target organisms. This is because gene sequences, both from the pest and from non-target species, have variations that allow inventors to construct a specific dsRNA that acts only on target organisms.

In particular, the inventors surprisingly developed dsRNA molecules-which are naturally non-toxic, non-allergenic, and non-virulent-that contain the synthetic sequences for the expression of dsRNAs that activate the silencing of transcripts of the genes *Trehalase II*, *Trehalose Transporter II*, and *Transcription Initiation Factor TFIID Subunit 1* of brown (*Euschistus heros*) and green-bellied (*Diceraeus melacanthus*) stink bugs, both pests in the agricultural production system, particularly in the soybean-corn production system, in addition to developing possible combinations between them, the concatamers. Namely, *dsTreha II*, *dsTreTrans II*, *dsTAF1* respectively and, as an example of a concatamer, *dsDrei*.

In a preferred embodiment of the invention, dsRNA molecules are defined according to any of the synthetic sequences for dsRNA expression with at least 50%, 60%, 70%, 80%, preferably 90% identity with any of the selected synthetic sequences from SEQ ID NO:1 to 6, or SEQ ID NO:13 to 15, or its variants and fragments.

The dsRNA molecules as described here comprise synthetic double-stranded RNA (dsRNA) sequences that act as inducers of the expression silencing mechanism of the target genes that have a corresponding sequence. These dsRNAs were obtained from the cloning of DNA fragments of the target genes in the pClone EZ vector, which is designed to contain two T7 promoters in opposite directions at the ends of the cloned fragments, thus allowing the transcription of two complementary strands of RNA that will give rise to the dsRNA.

In another embodiment of the invention, the small interfering RNA (siRNA) molecules are derived, modified or originate from the dsRNA molecules of the present invention.

In one of the invention's embodiments, the compounds that comprise at least one of the dsRNA molecules of the present invention do not have recombinant DNA and do not cause permanent modification of the genome of the plant in which it is used.

The compounds comprising the aforementioned dsRNA molecules of this invention are used for spraying crops, and can be applied alone or in combination with other existing technologies (such as chemical and biological pesticides, transgenic plants, among others), offering farmers a new sustainable tool, with a new mode of action, for the control and management of these pest insects.

In addition, the dsRNA molecules of this invention can also be used to generate transgenic plants or edited via genome editing tools.

In another embodiment of this invention, the method of obtaining dsRNA molecules comprises the steps of:
a) cloning in a cloning vector with instruction for the production of dsRNA for a target gene;
b) producing via fermentation, transcription *in vitro*, chemical synthesis, biochemical synthesis or the combination thereof of an unpurified dsRNA;
c) purifying the dsRNA molecules obtained.
d) formulating dsRNA molecules with carrier compositions containing chitosans, liposomes, gelatins, carbon nanotubes, carbon nanoparticles, nanofibers, clays, metals, modified clays, adjuvants, pheromones, biopolymers and others.

In an embodiment of this method, the target gene is *Trehalase II*, which acts on the hydrolysis of the carbohydrate trehalose, releasing two glucose molecules. In another embodiment, the target gene is *Trehalose Transporter II*, which is responsible for transporting trehalose synthesized in adipocytes to other tissues that need a carbon source, thus regulating trehalose levels in the hemolymph. Further, in yet another embodiment of the invention, the target gene is *Transcription Initiation Factor TFIID Subunit 1*, and it is a general transcription factor that acts on transcription initiation. In a preferred embodiment, step a) comprises the cloning in the cloning vector of any of the synthetic sequences SEQ ID NO: 1 through 6, or SEQ ID NO: 13 to 15.

In some embodiments of the invention, step (b) of the method described above is performed via the transformation receptor, such as fungi, yeasts, algae, plants or bacteria. More preferably, step (b) is performed via any selected microorganism from the group consisting of: *Pichia pastoris*, *Komagataella phaffii*, *Neurospora crassa*, *Saccharomices cerevisae*, *Corynebacterium glutamicum*, *Aspergillus nidulans*, *Saccharomyces* ssp., *Bacillus* ssp., *Chlamydomonas reinhardtii*, *Nicotiana* spp., *Saccharum* spp., *Amaranthus* spp.; *E. coli,* among others.

In a preferred embodiment of the invention, step (b) of the method described above, *in vitro* transcription of the dsRNA molecule of interest, is performed via host cell, preferably bacterium *E. coli* of the HT115 (DE3) strain, transformed with cloning vector of step (a), preferably the modified pClone EZ plasmid containing the sequences for the expression of the dsRNA of interest.

Another embodiment of the invention refers to plasmids that comprise the synthetic sequences of the genes *Trehalase II*, *Trehalose Transporter II and Transcription Initiation Factor TFIID Subunit 1*, which encode dsRNA sequences for silencing the genes of brown (*Euschistus heros*) and/or green-bellied (*Diceraeus melacanthus*) stink bugs. In a preferred embodiment of the invention, the plasmid comprises the synthetic sequences of genes as set forth in the SEQ ID NO: 1 to 6.

In an embodiment of the present invention, the compounds comprising the dsRNA molecules as described here can be used for the control of pest insects, preferably, the present invention discloses the method of pest insect control in an agricultural production system, which comprises: spray the compound comprising at least one of the dsRNA molecules in the plant so that the pest insect comes into contact with said dsRNA molecules. In a preferred embodiment of the invention, the pest insect control method is carried out in a soybean-corn system. In a more preferred embodiment, the insect pest is either brown stink bug (*Euschistus heros*) or green-bellied stink bug (*Diceraeus melacanthus*) or both.

The control of the pests *Euschistus heros* and *Diceraeus melacanthus* as described herein has great target specificity, high control efficiency and low environmental impact.

### EXAMPLES

The following examples, described in detail, serve to illustrate embodiments of this invention without, however, having any limitation character to the scope of protection thereof.

### OBTAINING dsRNA MOLECULES

*in vitro* manipulation of the coding sequence (CDS) of three brown stink bug genes, identified as follows:
Gene 1: compound 001 - *Trehalase II*, acts on the hydrolysis of the carbohydrate trehalose, releasing two glucose molecules.

A 410 bp region was selected, starting at position 1385 of the CDS to position 1795, which was used in the *in vitro* transcription of the double-stranded RNA (dsRNA) molecule against the *Trehalase II* (*dsTreha II*) gene. Below is the sequence of the region selected for expression of the synthetic gene, which encodes the dsRNA for silencing the *Trehalase II* gene of the brown stink bug and the green-bellied stink bug:

SEQ ID NO:1 and SEQ ID NO:2:
GGATGGAGATAAAGAAAAATTGTTCGTTGATATCAAATCAGCAGCGGAATCAGGATGGGATT
TCTCAACCAGGTGGTTTATACATAATGGCACTAACCATGGAAATATGTCTACTATCCACACT
CGTTACATTATACCAGTGGATTTGAATGCACTACTACACAGCAATGCAAAAATACTGTCTAG
TTGGTATAAAAAGTTTGGAAATAAAGATAAAGCTAAAAAATATGAATTACTATCAAAGGAAA
TTCTTGAAGGAATCGAAGAGATTCTATGGAATGATGAAGAAGGTATCTGGTTGGACTATGAT
ATTTTAAATGAAAAACCAAGAAATTATTTCTATCTTTCAAACTTTGTACCTTTCTGGACAAA
GTCGTACACATTTTCTTCAAAGCAAGCTGCAGCATTCG

Gene 2: compound 002 - *Trehalose Transporter II*, responsible for transporting trehalose synthesized in adipocytes to other tissues that require a carbon source, thus regulating trehalose levels in the hemolymph.

A region of 314 bp was selected, starting at position 1268 to position 1582, which was used in the transcription *in vitro* of the double-stranded RNA (dsRNA) molecule against the *Trehalose Transporter II* gene (*dsTreTran II*). Below is the sequence of the region selected for expression of the synthetic gene, which encodes the dsRNA for silencing the *Trehalose Transporter II* gene of the brown stink bug and the green-bellied stink bug:

SEQ ID NO:3 and SEQ ID NO:4
CGAAAATGATTATGATAAAAAAGAAGTGAATTCACAGCTTTATAGTCCACCCCTGGATACAA
ATGAAGGAAGCAATAGTAATTCAGGAGGACTCTTTAAACATGCTTCGACACAATGTTTTTTT
AACGATGTTATCGATAATGAAAAGGTAAAGAGTGGAGATAGAAGTGAAGCAGAGGAACCAAT
GTTTAGATTTTTATTGAAACCTGAAATGTACAAGCCAACATTACTCATGCTAGTTTGCAGTT
TCTTTGTTGTCGGACCTGGAGCTGACAGTATTTTAATTTATTTGAAGTTTTATTTGGACGAA
TTTA

Gene 3: compound 003 - *Transcription Initiation Factor TFIID Subunit 1*, is a general transcription factor that acts on transcription initiation.

A 425 bp region was selected, starting at position 3696 to position 4121, which was used in the transcription *in vitro* of the double-stranded RNA (dsRNA) molecule against the *Transcription Initiation Factor TFIID Subunit 1* gene (*dsTAF1*). Below is the sequence of the region selected for expression of the synthetic gene, which encodes the dsRNA for silencing the *Transcription Initiation Factor TFIID Subunit 1* gene of the brown stink bug and the green-bellied stink bug:

SEQ ID NO:5 and SEQ ID NO:6
AATCAGCCTGGTCGTGTTTTGAAAATTTATAGGACCTTTAGGGATTCTGAAGGGAAAGAATA
CACTCGATCTGAGGTTGTTAGGAAGCCAGCGGTTATCGATATGTATTTAAAGATTAGGAACA
CCAAGGACGAAGCGTTCATAAGACACTACGCAACTATGGACGATCAGCTCAAAGAGGAGATG
AAACGGGAAAAGAGGAGGCTTCAAGAGCAGCTGAGGAGGCTAAAGAGAAATCAGGAACGAGA
AAGAATCACCGGTGGTAGTAGTGGGCTATTTAATAAAACTCAGTCTCCTTTCTCTTTATTAT
CTACTCAAACTCTTGAAGGAGGTGAAACTAGAGATGGCTCACAATCTCCAACACCGAAGTCT
GGCTTCGTTAAGAATCGTCTACCTCCCCTCACTAACCCTACGACTCCTCCCAC

### COMBINATIONS OF dsRNA MOLECULES:

The following are sequences representing some of the possible combinations of the dsRNA molecules of the present invention:
SEQ ID NO:13 
AGGAGGACTCTTTAAACATGCTTCGACACAATGTTTTTTTAACGATGTTATCGATAATGAAA
AGGTAAAGAGTGGAGATAGAAGTGAAGCAGAGGAACCAATGTTTAGATTTTTATTGAAACCT
GAAATGTACAAGCCAACATTACTCATGCTAGTTTGCAGTTTCTTTGTTGTCGGACCTGGAGC
TGACAGTAACCAGGTGGTTTATACATAATGGCACTAACCATGGAAATATGTCTACTGAATGC
ACTACTACACAGCAATGAAGGAAATTCTTGAAGGAATCGAAGAGATTCGGTATCTGGTTGGA
CTATGAGGGAAAGAATACACTCGATCTGAGGTTGTTAGGAAGCCAGCGGTTATCGATATGTA
TTTAAAGATTAGGAACACCAAGGACGAAGCGTTCATAAGACACTACGCAACTATGGACGATG
AAACGGGAAAAGAGGAGGCTTCAAGAGCAGCTGAGGAGGCTAAAGAGGAATCACCGGTG
SEQ ID NO:14 
AGGGAAAGAATACACTCGATCTGAGGTTGTTAGGAAGCCAGCGGTTATCGATATGTATTTAA
AGATTAGGAACACCAAGGACGAAGCGTTCATAAGACACTACGCAACTATGGACGATAGGAGG
ACTCTTTAAACATGCTTCGACACAATGTTTTTTAACGATGTTATCGATAATGAAAAGGTAA
AGAGTGGAGATAGAAGTGAAGCAGAGGAACCAATGTTTAGATTTTTATTGAAACCTGAAATG
TACAAGCCAACATTACTCATGCTAGTTTGCAGTTTCTTTGTTGTCGGACCTGGAGCTGACAG
TAACCAGGTGGTTTATACATAATGGCACTAACCATGGAAATATGTCTACTGAATGCACTACT
ACACAGCAATGAAGGAAATTCTTGAAGGAATCGAAGAGATTCGGTATCTGGTTGGACTATGG
AAACGGAAAAGAGGAGGCTTCAAGAGCAGCTGAGGAGGCTAAAGAGGAATCACCGGTG
SEQ ID NO:15

AACCAGGTGGTTTATACATAATGGCACTAACCATGGAAATATGTCTACTGAATGCACTACTA
CACAGCAATGAAGGAAATTCTTGAAGGAATCGAAGAGATTCGGTATCTGGTTGGACTATGAG
GGAAAGAATACACTCGATCTGAGGTTGTTAGGAAGCCAGCGGTTATCGATATGTATTTAAAG
ATTAGGAACACCAAGGACGAAGCGTTCATAAGACACTACGCAACTATGGACGATAGGAGGAC
TCTTTAAACATGCTTCGACACAATGTTTTTTAACGATGTTATCGATAATGAAAAGGTAAAG
AGTGGAGATAGAAGTGAAGCAGAGGAACCAATGTTTAGATTTTTATTGAAACCTGAAATGTA
CAAGCCAACATTACTCATGCTAGTTTGCAGTTTCTTTGTTGTCGGACCTGGAGCTGACAGTG
AAACGGAAAAGAGGAGGCTTCAAGAGCAGCTGAGGAGGCTAAAGAGGAATCACCGGTG

### DESCRIPTION OF THE TRANSFORMATION RECEPTOR ORGANISM - E. COLI STRAIN HT115 (DE3)

The description of the bacterium and vector used for the production of dsRNA, as well as other relevant information, are described in the table below (Table 1).

**TABLE 1. ELEMENTS USED IN THE PROCESS OF OBTAINING THE COMPOUNDS OF THIS INVENTION.**

| Element | Description |
|---|---|
| Recipient microorganism | *E. coli* strain HT115 (DE3). Non-virulent, non-pathogenic and non-toxic. |
| Delivery microorganism | None. |
| Donor microorganism to recipient microorganism | None. |
| Plasmid pClone EZ Modified | Plasmid containing the synthetic sequences of the genes *Trehalase II, Trehalose Transporter II* and *Transcription Initiation Factor TFIID Subunit 1* that encode dsRNA sequences for gene silencing of brown stink bugs *(Euschistus heros)* and green-bellied *(Diceraeus melacanthus),* both insect pests in the agricultural production system. |
| Donor microorganism of the pClone EZ plasmid | *E. coli* |
| dsRNA Targets | Genes of brown *(Euschistus heros)* and green-bellied *(Diceraeus melacanthus)* stink bugs, with sequences complementary to those of the *Trehalase II, Trehalose Transporter II* and *Transcription Initiation Factor TFIID Subunit 1* genes resulting in their respective silencing. |
| Target Organisms | *Euschistus heros and Diceraeus melacanthus* |

### MODIFICATIONS MADE TO THE ORGANISM RECEIVING THE TRANSFORMATION - E. COLI STRAIN HT115 (DE3)

For the production of the dsRNA molecules that make up the compounds of the present invention, the techniques of gene cloning and heterologous expression in bacteria *E. coli* detailed below were used.

A specific fragment of the coding sequence of the target gene was amplified by polymerization chain reaction (PCR) using complementary DNA (cDNA) of the brown stink bug. For amplification via PCR, the primer oligonucleotides were designed to add the restriction enzyme *BsmBI* recognition sequences to the ends of the amplified product as shown in Table 2.

**TABLE 2. PRIMER OLIGONUCLEOTIDES USED IN THE CLONING OF TARGET GENE SEQUENCES IN THE MODIFIED EZ PCLONE VECTOR.**

| Oligo ID | Sequence 5' - 3' | TM (°C) | Amplicon (pb) |
|---|---|---|---|
| *dsTreha-II* F | gggCGTCTCgcgaatGGAATCAGGATGGGAT TT (SEQ ID NO:7) | 54 | 256 |
| *dsTreha-II R* | GggCGTCTCgagaccCCAACCAGATACCTTC TTCA (SEQ ID NO:8) | 54 | |
| *dsTreTran F* | GggCGTCTCgcgaatCACCCCTGGATACAAA TG (SEQ ID NO:9) | 54 | 219 |
| *dsTreTran R* | GggCGTCTCgagaccCAGGTCCGACAACAAA GA (SEQ ID NO:10) | 55 | |
| *dsTAF1 F* | GggCGTCTCgcgaatAATCAGCCTGGTCGTG TTTT (SEQ ID NO:11) | 56 | 425 |
| *dsTAF1 R* | GggCGTCTCgagaccGTGGAGGAGTCGTAG GGTT (SEQ ID NO:12) | 59 | |

| | | | |
|---|---|---|---|
| CGTCTC: Recognition site of the restriction enzyme BsmBI. | | | |

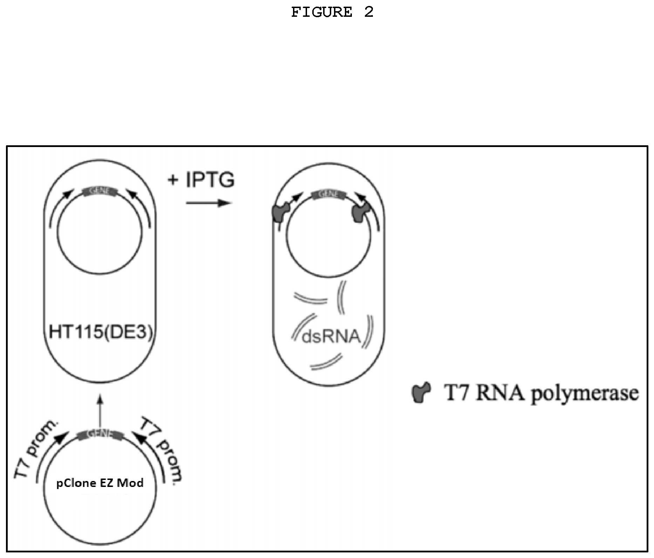

After checking specific amplification, both the PCR products and the modified pClone EZ vector, whose linear map containing the system for dsRNA production is shown in Figure 1a-c, were digested with the enzyme *BsmBI* and then the digested products were bound (Figure 1 - (a) to (d)). Then, the entire volume of the binding reaction was used for the transformation of *E. coli* HT115 (DE3) cells (Timmons; Court; Fire, 2001), via thermal shock. The *E. coli* HT115 strain was used for the production of dsRNA, since its RNase III activity is suppressed, allowing the accumulation of dsRNA within the cell without its degradation. The bacterium *E. coli* HT115 also has the gene encoding the enzyme RNA polymerase of the bacteriophage T7 that is under the control of an IPTG-inducible promoter (Isopropyl β-D-1-tiogalactopyranoside) (Figure 2).

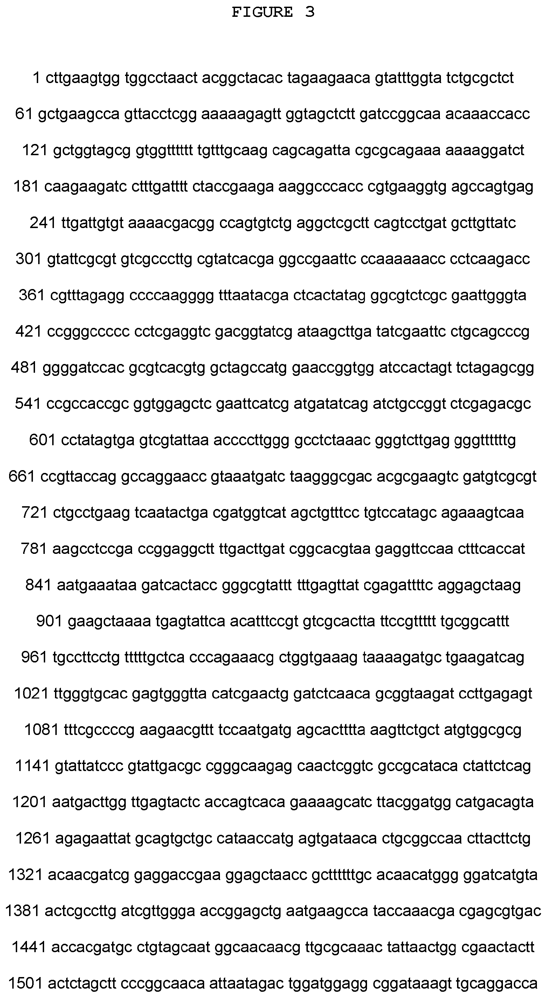

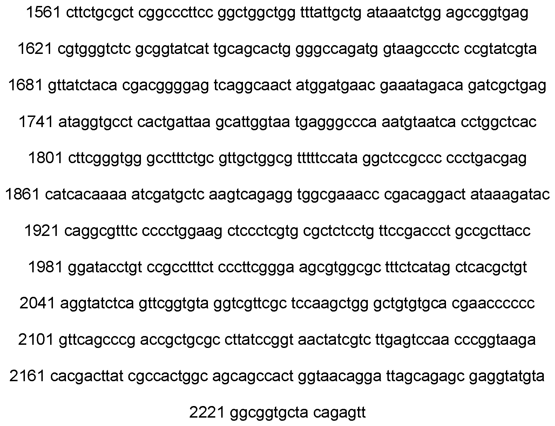

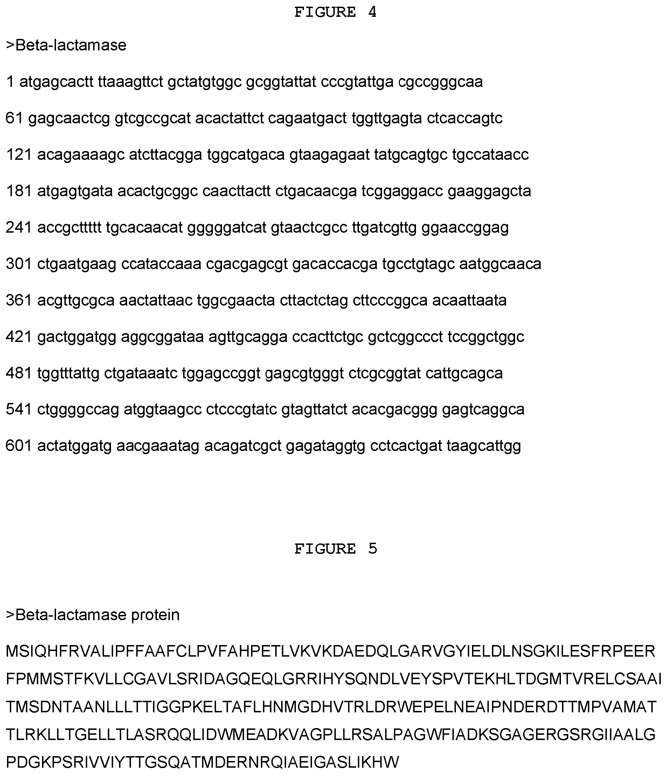

The complete nucleotide sequence of the modified pClone EZ plasmid is shown in Figure 3 and as established in SEQ ID NO:16, and the nucleotide sequence and protein of the Beta-Lactamase gene contained in the pClone EZ vector and conferring resistance to the antibiotic ampicillin is shown in Figures 4 and 5. The Beta-Lactamase gene protein is as established in SEQ ID NO:17. The partial sequence of the fragment amplified with M13 primers is shown in Figure 1c. The function and origin of the genetic elements present in the modified pClone EZ plasmid containing the dsRNA system inserted in *E. coli* HT115 (DE3) are presented in Table 3 below.

**TABLE 3. LOCATION AND FUNCTION OF THE GENETIC ELEMENTS PRESENT IN THE MODIFIED PCLONE EZ PLASMID CONTAINING THE SYSTEM FOR THE PRODUCTION OF dsRNA OF THE PRESENT INVENTION AND WHICH WAS INSERTED IN E. COLI STRAIN HT115 (DE3).**

| Component | Location | Function |
|---|---|---|
| Source | 1-2237 | organism="synthetic DNA construct" |
| | | mol_type="other DNA" |
| T7 terminator | 205-234 | label="T7 terminator" |
| | | note="T3Te terminator; phage T3 early transcription terminator" |
| primer_bind | 249-265 | label="M13 fwd" |
| | | note="M13 fwd; common sequencing primer, one of multiple similar variants" |
| misc_feature | 315-319 | label="Top I Site" |
| MCS | 320-691 | note="MCS" |
| T7 promoter | 383-401 | label="T7 promoter" |
| | | note="promoter for bacteriophage T7 RNA polymerase" |
| primer_bind | 434-450 | label="KS primer" |
| | | note="common sequencing primer, one of multiple similar variants" |
| primer_bind | Complement( 520-536) | label="SK primer" |
| | | note="common sequencing primer, one of multiple similar variants" |
| T7 promoter | Complement (601-619) | label="T7 promoter" |
| | | note="promoter for bacteriophage T7 RNA polymerase" |
| misc_feature | Complement( 692-703) | label="Top I Site" |
| | | |
| misc_featur e | Complement( 692-696) | label="Top I Site" |
| primer_bind | Complement( 746-762) | label="M13 rev" |
| | | note="M13 rev; common sequencing primer, one of multiple similar variants" |
| T7 terminator | 775-806 | label="T7 terminator" |
| | | note="tonB terminator; bidirectional E. coli tonB-P14 transcription terminator" |
| BLA promoter | 807-909 | label="promoter" |
| | | note="cat promoter; promoter of the E. coli cat gene" |
| BLA CDS | 910-1770 | codon_start=1 |
| | | gene="bla", product="beta-lactamase" |
| | | label="AmpR" |
| | | note="AmpR; confers resistance to ampicillin, carbenicillin, and related antibiotics" |
| terminator | 1794-1821 | label="terminator" |
| | | note="T7Te terminator; phage T7 early transcription terminator" |
| Replication Origin | 1833-183 | direction=LEFT |
| | | label="Ori" |
| | | note="ori; high-copy-number colE1pMB1pBR322pUC origin of replication |

### OBTAINING dsRNA MOLECULES:

After transformation with the pClone EZ vector recombined with the DNA fragments of the target genes, the bacterial cells were used to produce the preinoculums in 50 mL tubes containing 15 mL of TB medium (Terrific Broth, see Table 4) with the addition of the antibiotics tetracycline (12.5 µg/mL) and ampicillin (100 µg/mL) and incubated for 16 h at 37° C under 180 rpm agitation.

**TABLE 4. COMPOSITION OF THE TB (TERRIFIC BROTH) MEDIUM, USED IN THE FERMENTATION PROCESS FOR THE PRODUCTION OF dsRNA.**

| Medium | Compounds | Quantity |
|---|---|---|
| TB (Terrific Broth) | Tryptone | 12 g |
| | Yeast extract | 24 g |
| | Glycerol | 5 mL |
| | Monopotassium phosphate | 2.31 g |
| | Dipotassium phosphate | 12.54 g |
| | Water | 995 mL |

After this period, it was centrifuged at 9000 x *g* for 5 min, the supernatant was discarded and the pellet with bacterial cells was resuspended in 5 mL of new nutrient medium and incubated for 15 min at 37° C under 180 rpm agitation. 2 mL of each pre-inoculum for every 100 mL of TB medium were inoculated into a 1 L Erlenmeyer medium and incubated at 37° C under 180 rpm agitation until a DO ₆₀₀ nm of 0.4 was reached. Next, the expression of dsRNAs was induced by the addition of 2.5 mL of the lactose inducer (1mM) and maintained under the same conditions mentioned above for another 1 hour. Next, feedback was performed every hour with the addition of nutrient medium containing ampicillin and lactose, the amount to be added was fractionated into 1/4 of the initial volume, for a period of 4 hours, totaling 6 hours of fermentation process.

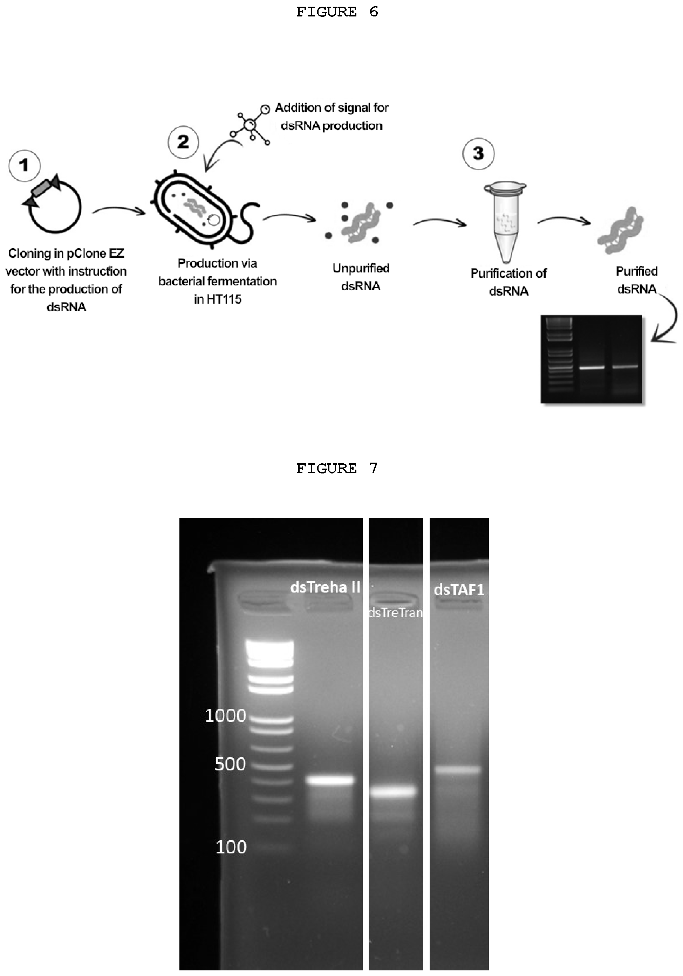

To finish, the bacterial suspension was collected by centrifugation at 9000 x *g* for 5 min, the supernatant was discarded and the *pellet* bacteria was used to obtain the dsRNA molecules. The general scheme of the process of production of dsRNA molecules via bacterial fermentation is presented in Figure 6.

After production, the purification of the dsRNA of compounds 001 to 003 of the present invention was composed of 3 steps: i) Extraction with Trizol; ii) Treatment with DNase I and RNAse A and iii) Purification with phenol:chloroform:isoamyl. The steps mentioned are detailed below:

### PURIFICATION OF dsRNA MOLECULES

### TRIZOL EXTRACTION

The cell wall and membrane of the bacteria contained in the pellets were dissolved by applying 800 µl of SDS 0.1% detergent in PBS 1X buffer and the tubes transferred to a water bath at 100° C for 2 min to promote the complete inactivation of the bacterial cells via total disruption of the plasma membrane. Then, 800 µl of Trizol reagent (Invitrogen, Carlsbad, CA, USA) was added and incubated at room temperature for 5 min. Then 200 µL of chloroform was added and incubated again at room temperature for 2 min, followed by centrifugation at 12000 x *g* for 10 min. The supernatant was removed and transferred to a new tube and 0.7 isopropanol volume was added, incubated at room temperature for 10 min and centrifuged again at 12000 x *g* for 10 min. After these procedures, two washes with 75% ethanol followed, and the pellet was then dried completely in *speed vac* and resuspended in 180 µl of nuclease-free water.

### TREATMENT WITH DNASE AND RNAse A

After the first extraction of nucleic acids including dsRNAs, the samples were treated with TURBO^{™} DNase (Ambion) and RNase A (Invitrogen) for removal of DNA from the parent microorganism and single-stranded RNA, as described by Ahn et al. (2019). The nucleic acids, including the dsRNAs from the first extraction with Trizol, were diluted in 180 µl of water and treated with 2 µL of TURBO^{™} DNAse and 20 µL of RNAse A and incubated for 1 h at 37° C. After the treatment, the purification stage was performed.

### PURIFICATION WITH PHENOL:CHLOROFORM:ISOAMYL

After treatment with DNase and RNase, the samples were purified according to the protocol of Ahn et al. (2019). In each of the samples, 200 µL of phenol:chloroform:isoamyl (25:24:1) was added, and after vigorous agitation, centrifugation at 12000 x g for 15 min followed. The upper phase was transferred to another tube and then 100 µL of NH4OAc (7.5M) and 100 µL of isopropanol were added, followed by a new centrifugation at 12000 x *g* for 45 min at 4° C. This was followed by two washes with 70% ethanol and centrifugation at 12000 x *g* for 10 min. The *pellet* was dried and resuspended in nuclease-free water.

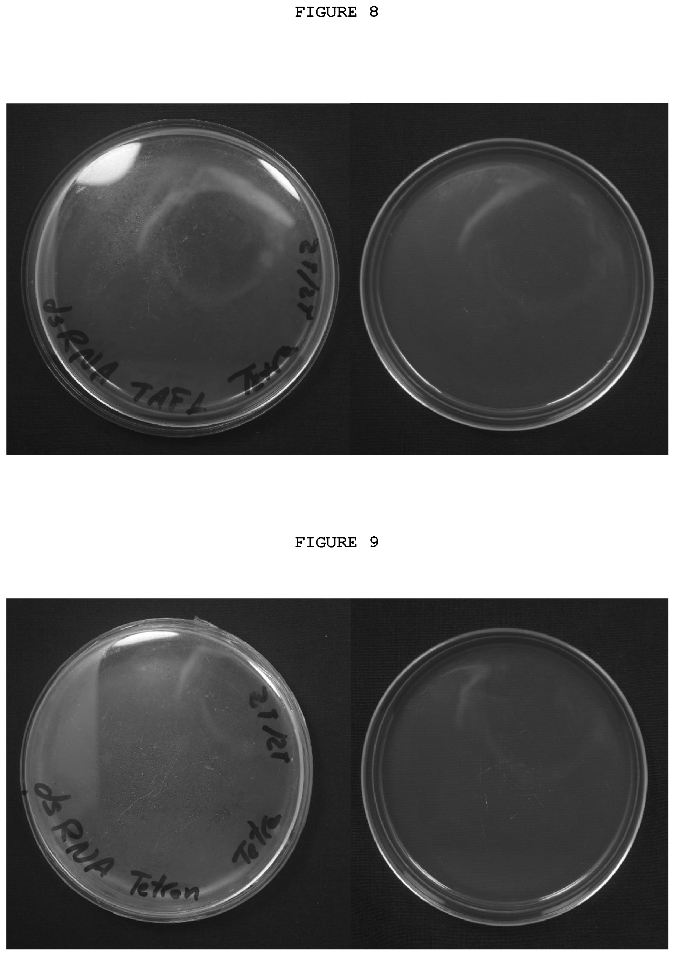

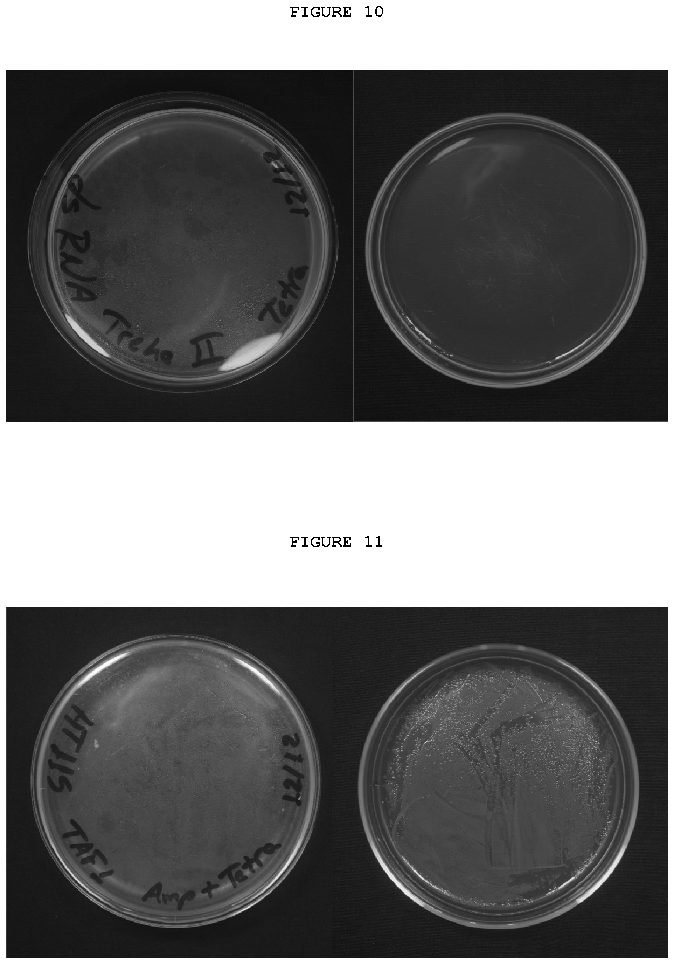

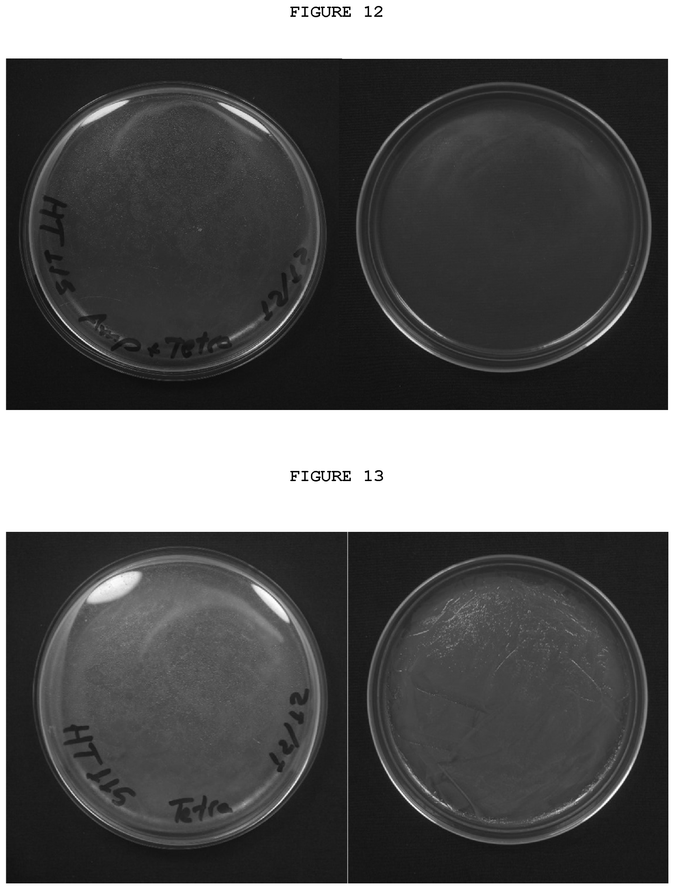

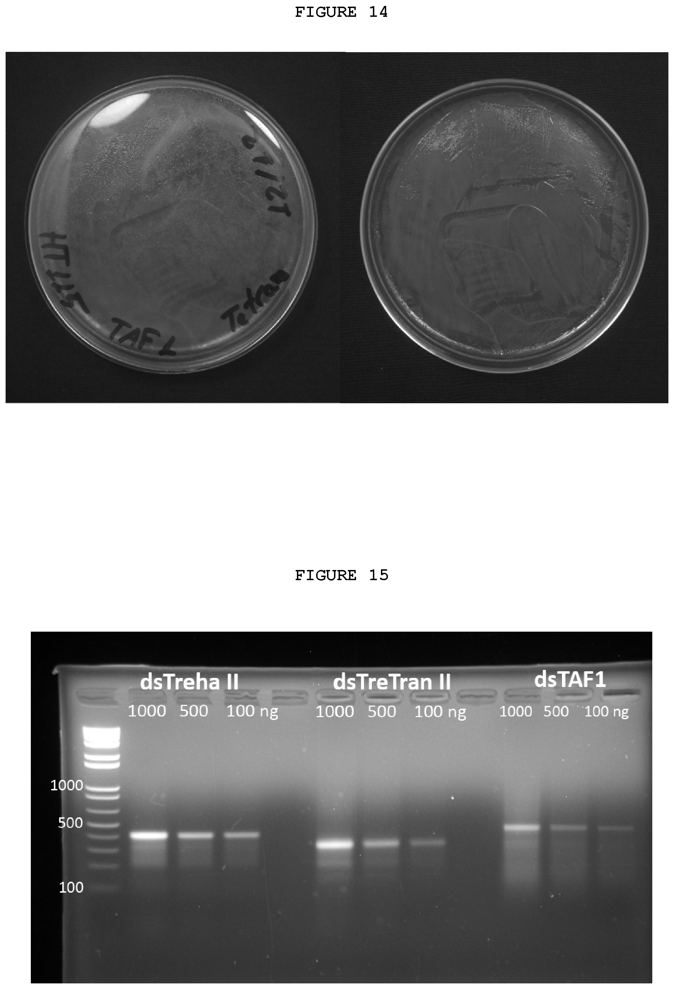

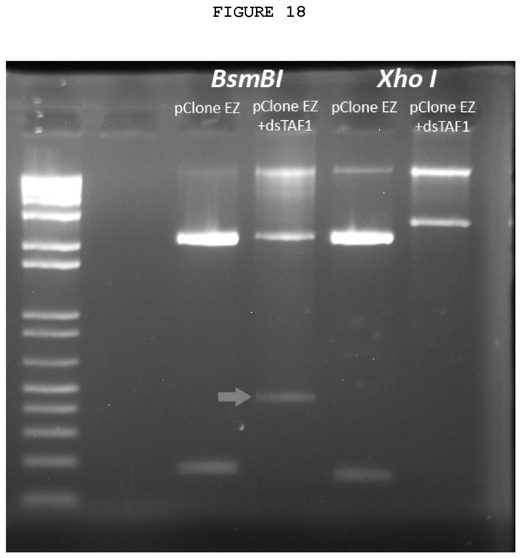

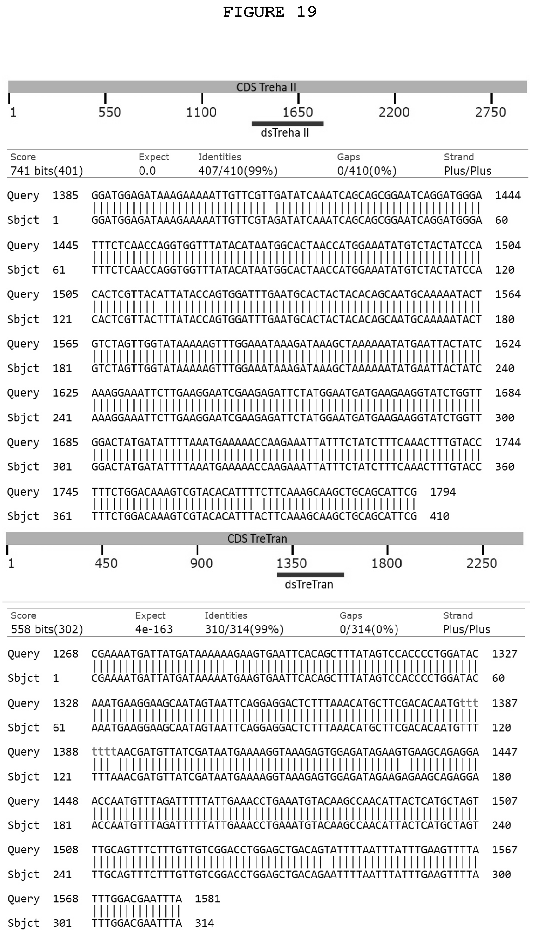

The samples were quantified in Nanodrop and their quality and absence of other nucleic acids was verified by electrophoresis in 1% agarose gel (Figure 7). Next, the analyses were performed to verify the presence/absence of recombinant DNA in compounds 001, 002 and 003, using the following approaches: (a) Checking the absence of live cells of *E. coli* in the dsRNA solution (see Figures 8 to 14); (b) PCR analysis to prove the absence of recombinant DNA molecules (see Figures 15 to 18) and (c) sequencing the product after purification (see Figure 19).

### PROOF OF THE ABSENCE OF RECOMBINANT DNA MOLECULES IN THE COMPOUNDS, THROUGH THE USE OF MOLECULAR METHODS

The analysis of the effectiveness of the purification process in eliminating any trace of bacteria and recombinant DNA in compounds 001, 002 and 003, after the production and purification process, was proven by three approaches:
- checking the absence of live *E. coli* cells in the dsRNA solution, by plating the solution of the compounds in plates containing LB culture medium with the antibiotics tetracycline (12.5 µg/mL) and ampicillin (100 µg/mL);
- PCR analysis to prove the absence of recombinant DNA molecules and
- Sequencing of the product after purification.

### CHECKING THE ABSENCE OF LIVE E. COLI CELLS IN THE COMPOUNDS

To analyze whether there is detection of bacteria in the dsRNA solution after the purification process, the final solution of the dsRNAs *TAF1*, *Treha II* and *TreTran II* was plated in LB medium, containing the antibiotic for the strain of *E. coli* HT115 that is resistant to the antibiotic tetracycline. As a positive control, an untransformed solution of *E. coli* HT115 was plated and transformed with the pClone EZ vector containing the dsRNAs before the purification process in LB medium with the antibiotic tetracycline. As a negative control, a solution of *E. coli* HT115 was plated untransformed in LB medium containing the antibiotics tetracycline and ampicillin (resistance to the latter antibiotic is provided by transformation with the pClone EZ vector).

The results of this analysis show the total absence of bacteria in the final solution in the compounds of the present invention, as shown in Figures 8 to 14.

The results of the cultivation of the final dsRNA solution of the compounds of the present invention in petri dishes (Figures 8 to 14) proved the absence of live bacteria in the dsRNA solution after the purification process.

### SEQUENCING THE COMPOUNDS IN THE FINAL dsRNA SOLUTION AFTER PURIFICATION

To verify the specificity of the nucleotide sequences of the dsRNAs *Treha II*, *TreTran II* and *TAF1* contained in compounds 001, 002 and 003 of the present invention, Sanger sequencing of the synthesized cDNA from the final dsRNA solution was performed. To this end, after purification and synthesis of cDNA from dsRNAs, the samples were submitted to sequencing using the BigDye^{®} Terminator v3.1 kit (Thermo Fisher Scientific, Waltham, MA, USA), in the automatic sequencer ABI PRISM Genetic Analyser^{<® 2} 3100} (Applied Biosystem, Foster City, CA, USA). The result of the sequencing demonstrated the specificity of the nucleotide sequences of the dsRNAs *Treha II*^{,} *TreTran II* and *TAF1* contained in the compounds of the present invention as shown in Figure 19.

### EFFICACY TESTING - BIOASSAYS

### Combined sequence test with part of dsTreTrans, dsTreha II, dsTAF1, and dsDrei

Bioassay tests were carried out to prove the efficacy of the dsRNA molecules of this invention.

In a first test, the sequence *dsDrei* (*dsTreTrans* + *dsTreha II* + *dsTAF1*) was tested as follows:

SEQ ID NO:13
AGGAGGACTCTTTAAACATGCTTCGACACAATGTTTTTTAACGATGTTATCGATAATGAAA
AGGTAAAGAGTGGAGATAGAAGTGAAGCAGAGGAACCAATGTTTAGATTTTTATTGAAACCT
GAAATGTACAAGCCAACATTACTCATGCTAGTTTGCAGTTTCTTTGTTGTCGGACCTGGAGC
TGACAGTAACCAGGTGGTTTATACATAATGGCACTAACCATGGAAATATGTCTACTGAATGC
ACTACTACACAGCAATGAAGGAAATTCTTGAAGGAATCGAAGAGATTCGGTATCTGGTTGGA
CTATGAGGGAAAGAATACACTCGATCTGAGGTTGTTAGGAAGCCAGCGGTTATCGATATGTA
TTTAAAGATTAGGAACACCAAGGACGAAGCGTTCATAAGACACTACGCAACTATGGACGATG
AAACGGGAAAAGAGGAGGCTTCAAGAGCAGCTGAGGAGGCTAAAGAGGAATCACCGGTG

The test was performed using microinjection as an exposure method. Treatment was:
4 dsRNAs (*dsDrei*, *dsTAF1*, *dsTreha II* and *dsTreTran*) at a dose of 28 ng dsRNA/mg of stink bug weight; 1 water (control of microinjection); 1 control without microinjection (control of the health of the stink bug sample).

There were 4 replicates per treatment and 10 insects per replicate, i.e., n=40.

After microinjection, the adult insects of each replication were kept in Gerbox boxes (11x11x3.5 cm) with standard feeding for 20 days.

Mortality and oviposition were assessed up to 20 days after application. ANOVA statistical analysis and comparison of Tukey averages (P<0.05) were used.

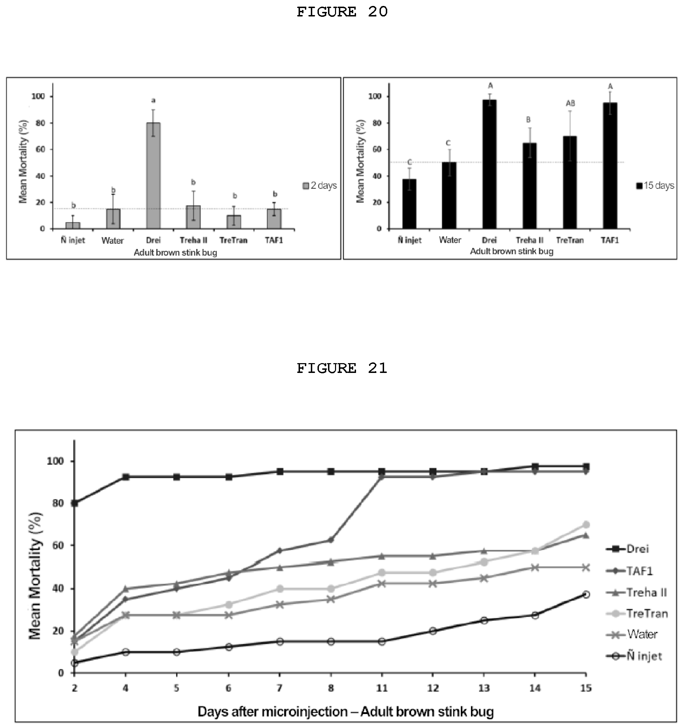

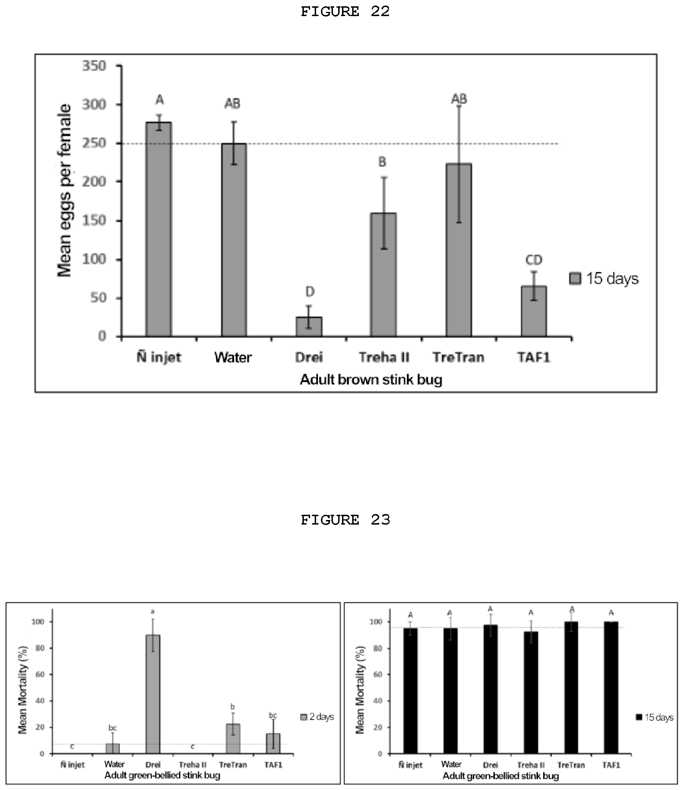

Only 48 h after microinjection with *dsDrei* did we observe mortality greater than 80 and 90% in brown and green-bellied stink bugs, respectively. Action similar to the shock effect of chemical insecticides as shown in Figures 20 and 23.

We observed a mortality of 95% in the brown stink bug 15 days after microinjection with dsTAF1 (Figure 20, 21).

We observed a mortality of 65 and 70%, respectively, in the brown stink bug, 15 days after microinjection with *dsTreha II* and *TreTran II* respectively (Figure 20, 21).

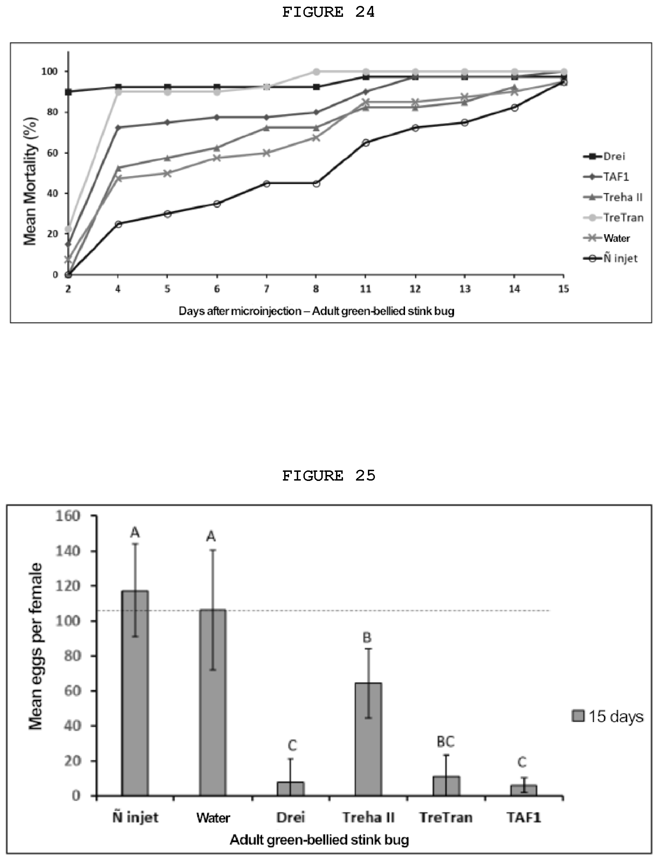

In the green-bellied stink bug, we observed an exclusive mortality of 90% on the second day after microinjection with *dsDrei*, indicating a specific effect of this molecule on the stink bug (Figure 23, 24).

Also, the effect of dsRNA on oviposition is surprising and impactful, since a drastic reduction in the amount of eggs was observed for all dsRNAs tested. So in addition to causing significant mortality, the microinjection of these dsRNAs also greatly reduced the population of survivors (Figures 22 and 25).

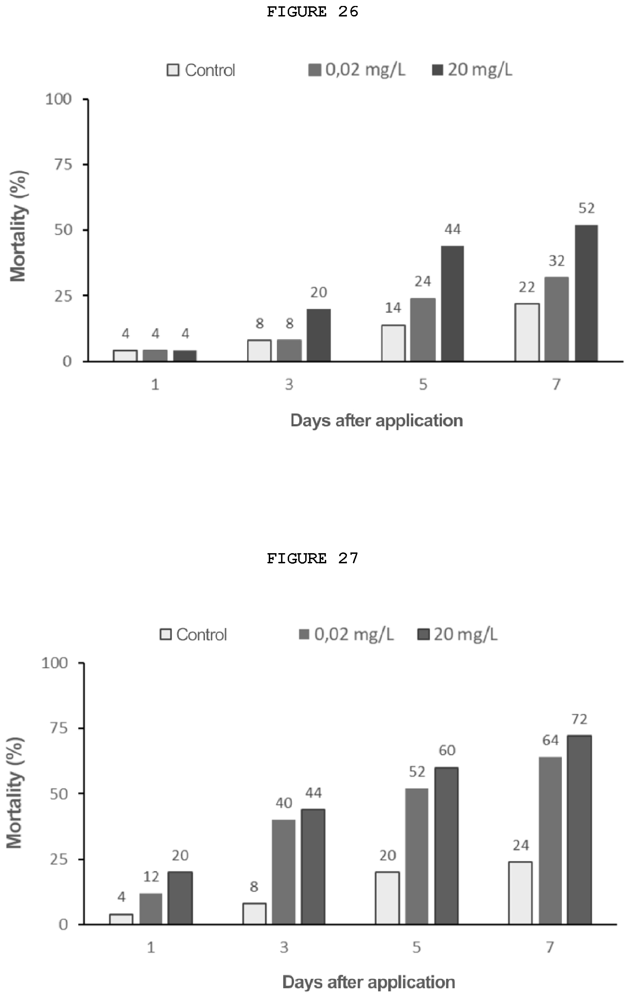

For the dsDrei naked spray tests, spraying was performed with the aid of a Potter's tower (Burkard Manufacturing, Rickmansworth, Herts, England) calibrated at a pressure of 10 psi (68.95 kPa), using a volume of 2 mL of solution in each spray, obtaining an average deposition of wet residue of 1.50 mg/cm². The treatments tested were: untreated control, aqueous solution of dsRNA at 0.02 mg/L and 20 mg/L of *dsDrei naked*, with 4 replicates per treatment and 10 insects per replicate, n = 40. After spraying, the adults were kept in Gerbox boxes (11x11x3.5 cm) with standard feeding for 7 days. Mortality assessments were made daily up to 7 days after spraying. The percentage of mortality of brown and green-bellied stink bugs was calculated. Based on Figures 26 and 27, it was evidenced that the dsDrei in the naked version tested presented mortality of up to 50% approximately for the brown stink bug and up to 70% approximately for the green-bellied stink bug, after 7 days of spraying.

### TOXICITY TESTS

To evaluate the toxicity of the putative proteins, generated by the dsRNAs of the present invention - compounds 001, 002 and 003 and the concatemer described here, the ToxinPred2 (https://webs.iiitd.edu.in/raghava/toxinpred2/ blast_action.php) software was used. The ToxinPred2 software was developed to predict protein toxicity (Gupta et al., 2013; Sharma et al., 2022). This is the latest version of the software with an improved algorithm and an expanded database.

The software was developed by researchers at the Bioinformatics Center of CSIR - Institute of Microbial Technology, Chandigarh, India. It is known to a person skilled in the art, since it is widely used in the literature to predict toxicity. It utilizes a wide range of information and forecasting techniques that include machine learning, BLAST, and MERCI. The models were trained on a large dataset that contains 8233 toxic and 8233 non-toxic.

The method was tested and evaluated on three curated datasets from SwissProt. To provide unbiased evaluation, internal validation is performed on 80% of the data and external validation on the remaining 20%. The techniques used to predict protein toxicity are: (a) Similarity based on the Basic Local Alignment Search Tool, (2) Motif-EmeRging with motif search based on Class Identification, and (3) Prediction Models.

Techniques based on similarity and motifs achieve a high probability of correct prediction with low sensitivity/coverage, while models based on machine learning techniques have achieved sensitivity and specificity of equilibrium with high accuracy. The algorithm involves a hybrid method of analysis, which combines all three approaches and achieves a maximum area under the receiver operating characteristic curve of around 0.99 with a Matthews correlation coefficient (MCC) of 0.91 in the validation dataset. In addition, the software uses models in alternative and realistic data sets. It is a method developed to predict the toxicity of proteins regardless of their source of origin.

The results of the toxicity tests can be seen in tables 5 and 6 below:

**TABLE 5. TOXICITY OF THE HYPOTHETICAL PEPTIDES GENERATED BY THE dsRNAs Treha II, Tretran II AND TAF1.**

| Legend: T1 to T5 = peptides generated by dsRNA *Treha II.* | | |
|---|---|---|
| TT1 to TT3 = peptides generated by dsRNA *TreTran II.* | | |
| TF1 to TF3 = peptides generated by dsRNA *TAF1.* | | |
| **ToxinPred2** | | |
| **Protein Code** | **Occurrence** | **Prediction** |
| T1 | No hits found | Non-toxin |
| T2 | No hits found | Non-toxin |
| T3 | No hits found | Non-toxin |
| T4 | No hits found | Non-toxin |
| T5 | No hits found | Non-toxin |
| TT1 | No hits found | Non-toxin |
| TT2 | No hits found | Non-toxin |
| TT3 | No hits found | Non-toxin |
| TF1 | No hits found | Non-toxin |
| TF2 | No hits found | Non-toxin |
| TF3 | No hits found | Non-toxin |

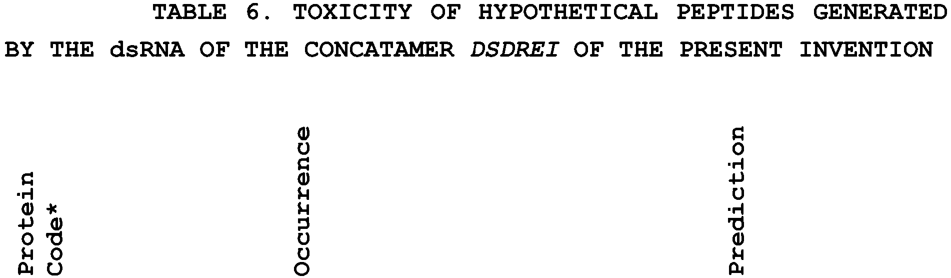

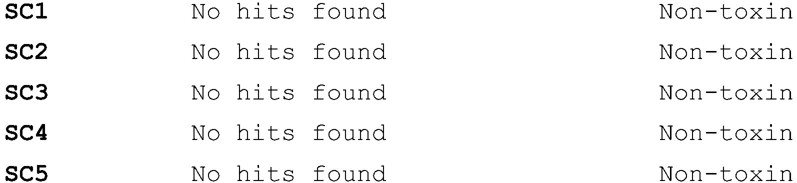

### ALLERGENICITY TESTING

It is known that dsRNA sequences are not translated into proteins (Liu et al., 2015). This information reinforces that dsRNA-based products are unlikely to cause immune responses in humans and animals. This is corroborated when it is observed that the immune response mechanism begins with protein antigens. The immune response mechanisms that trigger the allergic condition are described in detail in articles published in Nature Medicine (Akdis, 2012) (Impact factor 87.241) and in Clinical and Experimental Allergy (Scheurer; All; Vieths, 2015) (Impact factor 5.018).

Briefly, the mechanisms involved include the perception of the allergen (5-100 kDa proteins) by specific memory T and B cells and the production of specific antibody isotypes to trigger the inflammatory immune response, as well as the activation, tissue migration and degranulation of mast cells, basophils and eosinophils, which will release reactive substances such as, for example, histamine. As mentioned above, most allergens are relatively small proteins or glycoproteins that in most cases are water-soluble (Akdis, 2012; Scheurer; All; Vieths, 2015).

Therefore, it is necessary that some intrinsic biochemical characteristics of the protein structure are present to trigger immune responses, which does not apply to dsRNAs, as these are not translated into proteins. In addition, the allergic condition is dependent not only on the presence of the protein allergen, but is also determined by additional factors, such as the amount and duration of exposure to the immune system (Akdis, 2012; Scheurer; All; Vieths, 2015). According to Das and Sherif (2020), the residual effect of an encapsulated dsRNA after application does not exceed 30 days, showing a very short shelf life. However, dsRNA in its Naked version can be degraded in a matter of hours or minutes, depending on its composition, size, and environmental conditions to which it is exposed, such as solar radiation and temperature, which can further diminish its effect after application (Bachman et al., 2020).

Given the importance of protein structure to assess allergenicity, according to the aforementioned authors, the classification of allergens is given into structural groups, this approach facilitates the assessment of risk related to cross-reactivity. Cross-reaction refers to the same allergic response triggered by different allergens, but with very similar structural components. In other words, it is when the immune system reacts against proteins with similar sequences in the same way. Therefore, the similarity between proteins can facilitate the identification of new allergens.

Because allergenicity is a very complex and dynamic topic, it is constantly being studied. Data obtained are annually updated, compiled and deposited in several specific databases. Facilitating access to information regarding allergenicity and cross-reaction between proteins of different species. While there are several databases to assess whether or not a protein may be potentially allergenic, some banks are more robust and curated than others.

Among the most reliable databases are AllergenOnline (Sircar et al., 2014) and AllerTOP v2.0 (Dimitrov et al., 2013). AllergenOnline is considered by researchers at the Swiss Institute of Allergy and Asthma Research (SIAF), an institute associated with the University of Zurich, Switzerland, to be one of the main allergen banks available (Sircar et al., 2014). SIAF is a member of national and international organizations, such as the European Academy of Allergy and Clinical Immunology (EAACI). SIAF focuses on patient-relevant human immunology and cell biology, with a number of research projects on immunological regulatory aspects and particularly allergen tolerance, cell regulation and tissue barriers, methods for the detection of tissue barriers, regulatory aspects of noncoding DNA, and vaccine development.

The AllergenOnline database provides access to a peer-reviewed allergen list and a searchable sequence database intended for the identification of proteins that may present a potential risk of allergenic cross-reactivity. It has 2233 allergenic sequences from 912 taxonomic groups, and is updated annually. This bank was developed to help in the evaluation of the safety of proteins that can be introduced into food through genetic engineering or through food processing methods. The goal is to identify proteins that may require additional testing, such as serum IgE binding, histamine release by basophils, or in vivo challenge to assess potential crossreactivities.

The second highly robust database used to check the evaluation of the allergenicity of the sequences was AllerTOP v. 2.0 (2013). This second database uses a method based on the automatic transformation of cross-covariance (ACC) of protein sequences into uniform vectors of equal length. ACC is a protein sequence mining method developed by Wold et al. (1993). It has been applied to quantitative studies of structureactivity relationships (QSAR) of peptides with different lengths. In this bank, the main properties of amino acids are represented by five descriptors, originally derived from Venkatarajan and Braun (2001).

The descriptors used are: hydrophobicity of amino acids, molecular size, propensity to helice-forming, relative abundance of amino acids, and propensity to β-chain formation. In addition, proteins are classified by the k-nearest neighbor algorithm (kNN, k=1) based on a training set containing 2427 known allergens from different species.

Although dsRNAs are not translated into proteins as previously mentioned, an in-depth study was carried out on the allergenicity potential of putative proteins that could be generated from the dsRNA sequences of the present invention. The proteins were evaluated from the 2 banks mentioned above.

Tables 7 and 8 below present the results of the allergenicity tests.

**TABLE 7. ALLERGENICITY OF HYPOTHETICAL PEPTIDES GENERATED BY dsRNAS Treha II, Tretran II AND Taf1.**

| Legend**:** RC = cross-reaction. | | | | | |
|---|---|---|---|---|---|
| T1 to T5 = peptides generated by dsRNA *Treha II*. | | | | | |
| TT1 to TT3 = peptides generated by dsRNA *TreTran II*. | | | | | |
| TF1 to TF3 = peptides generated by dsRN*A TAF1*. | | | | | |
| **Protein code** | **AllergenOnline** | **% identity/YYY** | **AllerTOP v. 2.0** | **% identity/YYY** | **RC** |
| T1 | Nonallergenic | - | Non-allergenic | - | No |
| T2 | Non-allergenic | - | Non-allergenic | - | No |
| T3 | Non-allergenic | - | Non-allergenic | - | No |
| T4 | Non-allergenic | **-** | Allergenic | 27.08/48 | No |
| T5 | Non-allergenic | 44.4/18 | Allergenic | 29,03/30 | No |
| TT1 | Non-allergenic | 36,7/30-27,7/47-42.1/19 | Non-allergenic | - | No |
| TT2 | Non-allergenic | 36.6/41 | Non-allergenic | - | No |
| TT3 | Non-allergenic | 34,5/29 | Non-allergenic | - | No |
| TF1 | Non-allergenic | 19,4/98 | Non-allergenic | - | No |
| TF2 | Non-allergenic | - | Non-allergenic | - | No |
| TF3 | Non-allergenic | 39,1/23 - 37,9/29 | Non-allergenic | - | No |

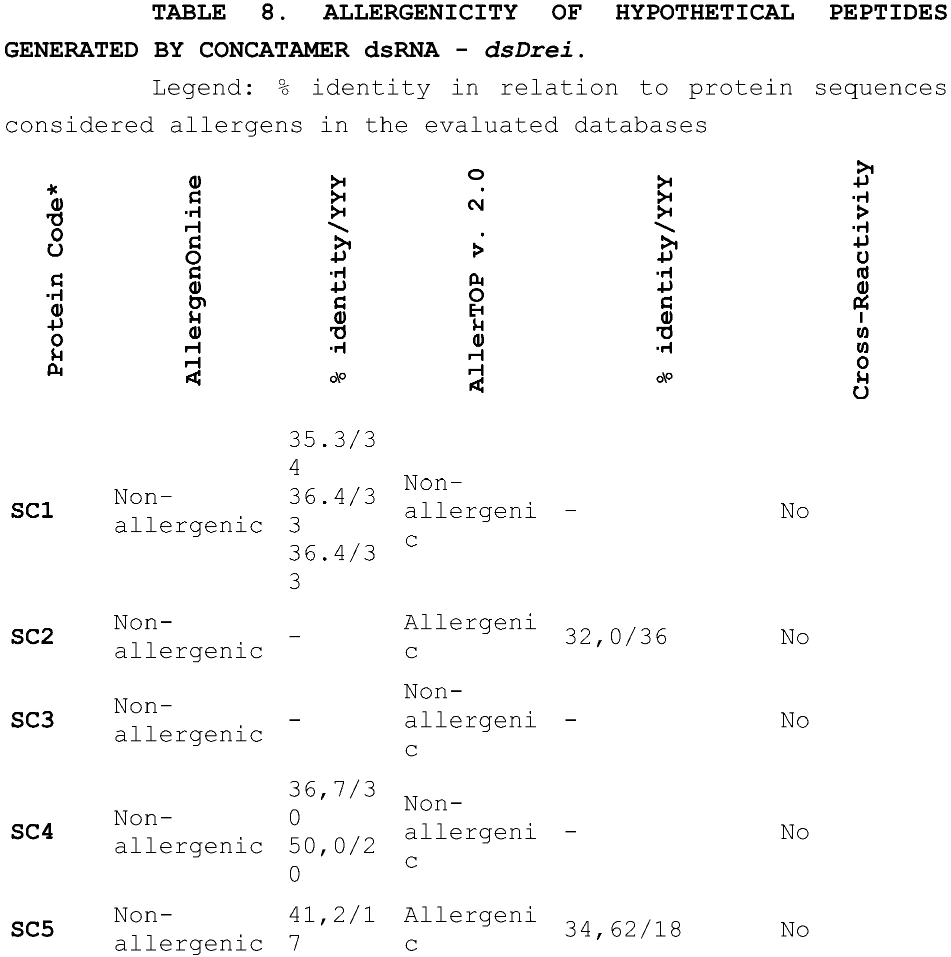

It is clear from the data mentioned above that the dsRNAs related to compounds 001, 002 and 003 and to the concatemer of the present invention do not present allergenicity and toxicity. Complying with the guidelines of the CODEX Alimentarius (2003).

It should be understood that the embodiments described above are merely illustrative and that several modifications can be made by a person skilled in the art without straying from the scope of this invention. Consequently, the present invention should not be regarded as being limited to the examplary embodiments described in this application. In addition, this disclosure may include material not currently claimed but which may be claimed in the future in combination with or separately from the characteristics claimed herein.

## Claims

1. dsRNA molecules **characterized in that** they contain the synthetic sequences for the expression of dsRNAs that activate the silencing of the transcripts of genes selected from *Trehalase II (TrehaII)*, *Trehalose Transporter II (TreTran II)*, *Transcription Initiation Factor TFIID Subunit 1 (TAF1)* or at least one concatamer comprising a combination of these sequences.

2. dsRNA molecules according to claim 1, **characterized in that** they are defined according to: synthetic sequences for dsRNA expression with at least 50%, 60%, 70%, 80%, preferably 90% identity with any of the selected synthetic sequences from SEQ ID IN: 1 through 6, or SEQ ID NO: 13 to 15 or their variants and fragments.

3. Small interfering RNA (siRNA) molecules **characterized in that** they are derived, modified, or originate from dsRNA molecules as defined in either of claims 1 or 2.

4. Compound **characterized by** comprising at least one dsRNA molecule as defined in either of claims 1 or 2.

5. Method of obtaining dsRNA molecules **characterized by** comprising the steps of:
a. cloning in cloning vector with instruction for production of dsRNA for a target gene;
b. producing via fermentation, transcription *in vitro*, chemical synthesis, biochemical synthesis or the combination thereof of an unpurified dsRNA;
c. purifying the dsRNA molecules obtained.

6. Method of obtaining dsRNA molecules according to claim 5, **characterized by** step (a) understanding cloning in the cloning vector of synthetic sequences for the expression of dsRNAs that activate the silencing of gene transcripts *Trehalase II (TrehaII)*, *Trehalose Transporter II (TreTran II)* and *Transcription Initiation Factor TFIID Subunit 1 (TAF1)* or at least one concatamer comprising a combination between said sequences.

7. Method of obtaining dsRNA molecules according to either of claims 5 or 6, **characterized by** step (a) comprising cloning in the cloning vector of any of the synthetic sequences selected from SEQ ID NO: 1 to 6, or SEQ ID NO: 13 to 15.

8. Plasmid **characterized by** comprising at least one of the synthetic sequences of genes encoding dsRNA sequences for silencing target genes of insect pests.

9. Plasmid according to claim 8, **characterized by** comprising at least one of the synthetic sequences of the genes *Trehalase II*, *Trehalose Transporter II*, *Transcription Initiation Factor TFIID Subunit 1,* that encode dsRNA sequences for gene silencing of brown (*Euschistus heros*) and/or green-bellied (*Diceraeus melacanthus*) genes.

10. Plasmid according to either of claims 8 or 9, **characterized by** comprising the synthetic gene sequences as set forth in SEQ ID NO:1 to 6.

11. Plasmid according to any of claims 8 to 10, **characterized by** comprising SEQ ID NO:16.

12. Host cell **characterized by** comprising the plasmid as defined in any of claims 8 to 11.

13. Host cell according to claim 12, **characterized in that** said cell is from a bacterium.

14. Method of pest control in an agricultural production system **characterized by** comprising spraying the compound as defined in claim 4 on pest insects in such a way that they come into contact with at least one of the aforementioned dsRNA molecules as defined in any of claims 1 to 2.

15. Method according to claim 14, **characterized in that** the agricultural production system is the soybean-corn production system.

16. Method according to claims 14 or 15, **characterized in that** the insect pest is either brown stink bug (*Euschistus heros*) or green-bellied stink bug (*Diceraeus melacanthus*) or both.
